(19) Europäisches Patentamt
European Patent Office
Office européen des brevets

(11) **EP 0 560 630 B1**

## (12) EUROPEAN PATENT SPECIFICATION

(45) Date of publication and mention
of the grant of the patent:
**18.11.1998 Bulletin 1998/47**

(51) Int. Cl.$^6$: **B32B 27/34**, B32B 27/40,
B32B 27/36, B32B 27/12,
B32B 5/26

(21) Application number: **93301908.5**

(22) Date of filing: **12.03.1993**

(54) **Bicomponent polymeric films containing block poly (ether-co-amides)**

Polymere Bikomponentenfilme, enthaltend Blockpoly(ether-co-amide)

Films polymères à deux composants contenant des poly(ether-co-amides) séquencés

(84) Designated Contracting States:
**AT CH DE DK ES FR GB IT LI**

(30) Priority: **13.03.1992 US 850537**

(43) Date of publication of application:
**15.09.1993 Bulletin 1993/37**

(73) Proprietor: **McNEIL-PPC, INC.
Milltown New Jersey 08850 (US)**

(72) Inventors:
• **Cashaw, Alan G.
Brick, NJ 08724 (US)**
• **Dabi, Shmuel
Highland Park, NJ 08905 (US)**

• **Johnson, Bruce C.
Whiting, NJ 08759 (US)**
• **Persinko, Mark M.
Bridgewater, NJ 08807 (US)**

(74) Representative:
**Mercer, Christopher Paul et al
Carpmaels & Ransford
43, Bloomsbury Square
London WC1A 2RA (GB)**

(56) References cited:
**EP-A- 0 476 963          US-A- 4 635 624
US-A- 4 937 134**

## Description

The present invention relates to moisture-permeable, nonporous bicomponent polymeric films. More specifically, the invention relates to bicomponent films comprising two major layers, one of said major layers comprising a hydrophilic block poly(ether-co-amide) as one of its components and the other of said major layers comprising a generally hydrophobic polymer having certain specifically defined properties to be described hereinafter. The invention also relates to laminated structures comprising the bicomponent polymeric films as well as to articles, such as surgical gowns, patient drapes, protective garments and the like made from the laminated structures.

Polymeric films have been used for a considerable period of time in the manufacture of a variety of surgical, health and consumer care products. Among such products are surgical gowns and patient drapes, occlusive wound dressings and absorbent products such as sanitary napkins, disposable diapers for infants and incontinence garments for adults.

To be useful as components of such surgical and health care products, a polymeric film must be substantially nonporous, i.e. it must function as a barrier to the passage of liquids such as water, aqueous based irrigating liquids, aqueous alcohol solutions and body fluids such as blood, urine and the like. In addition, it is highly advantageous that such films be "breathable", i.e. they should readily permit the passage therethrough of water vapor. In other words, it is advantageous that the films have a high moisture vapor transmission rate (MVTR). For example, a wound dressing comprising a polymeric film having a high MVTR will allow water vapor from the wound exudate to pass therethrough. As another example, a surgical gown which utilizes a high MVTR polymeric film as one of its structural components allows perspiration to pass therethrough in the form of water vapor, thus making the gown considerably more comfortable to wear. Protective garments, e.g., pants, jackets and suits designed to protect the user from dust particles and the other contaminants encountered in commercial and industrial activities, can likewise be made more comfortable to wear when polymeric films having high MVTR's are used as structural components thereof.

EP-A-0 476 963 discloses polymeric blends comprising a mixture of a) a hydrophilic block poly(ether-co-amide) containing between about 20 percent and about 80 percent by weight of poly(ethylene glycol) blocks, and b) a hydrophobic polymer. The hydrophobic polymer may be a block poly(ether-co-amide) containing no poly(ethylene glycol) blocks, a polyamide, a polyester or a polyurethane. The polymeric blends disclosed in EP-A-0 476 963 can be melt processed using conventional extrusion or casting techniques to prepare nonporous, breathable films which exhibit a high MVTR and have a wet tensile strength which is greater than the wet tensile strength exhibited by a film prepared from the hydrophilic polymer alone.

Films prepared from the polymeric blends disclosed in EP-A-0 476 963 can be bonded, e.g., using heat bonding techniques or glue, to fabric backings to make film/fabric laminates. These film/fabric laminates may then be converted into disposable surgical gowns and patient drapes for use in the operating room. Such gowns and drapes are routinely contacted by aqueous based materials, e.g., water, saline solutions, aqueous isopropanol solutions, blood and the like body fluids, during medical or surgical procedures. The films disclosed in EP-A-0 476 963 however, tend to have reduced tensile strength when contacted by aqueous alcohol solutions, e.g., 70% aqueous iso-propanol, commonly found in operating rooms, physician offices and other medical facilities. This is especially true in those instances where the surgical gown or patient drape is stressed when wet. Such stressing would occur, for example, in the elbow area of a surgical gown (due to the bending of the wearer's elbow) or in the front portion of the gown (which tends to come into frictional contact with the edge of the operating table or other equipment used during surgery). Thus, it would be desirable to provide moisture vapor-permeable, nonporous films which provide resistance to aqueous alcohol solutions.

In accordance with the present invention, there is provided a nonporous, breathable bicomponent film which comprises two major layers of polymeric materials as defined in claim 1. These two major layers are normally co-extensive in length and width but, as will be seen, have different thicknesses. The second major layer of polymeric material provides resistance to aqueous alcohol solutions.

The first of the two major layers of polymeric material comprising the bicomponent film of the present invention includes a) a hydrophilic block poly(ether-co-amide) which contains between 20 and 80 percent by weight of polyethylene glycol (PEG) blocks and b) a hydrophobic polymer selected from a block poly(ether-co-amide) containing essentially no PEG blocks, a polyamide, a polyester or a polyurethane. The hydrophilic block copolymer constitutes between 20 to 90 weight percent of the polymeric components of the first major layer and the hydrophobic polymer constitutes the balance of the polymeric components. The first major layer may contain minor amounts of other additives, such as anti-oxidants, processing aids, pigments, etc., which are not film forming.

The second of the two major layers of polymeric material comprising the bicomponent film of the present invention includes a polymer or mixture of polymers, a thin film (i.e. a film having a thickness of approximately 25.4 $\mu$m (1 mil)) of which has certain defined characteristics relating to moisture vapor transmission rate, equilibrium water absorption and equilibrium 70% aqueous isopropanol absorption. Specifically, in order for a polymer or mixture of polymers to be useful in the second major layer, a film made from said polymer or mixture of polymers must, when tested at a thickness of 25.4 $\mu$m (1 mil), have the following properties:

a) an MVTR of greater than 1,000 g/sq.m/day of water as measured by the gravimetric test method set forth in ASTM E-96 (Dessicant Method);

b) an equilibrium water absorption of less than 25% by weight as determined in accordance with the test method to be described hereinafter; and

c) a 70% aqueous iso-propyl alcohol absorption, measured in accordance with the test method to be described hereinafter, of less than about 50%.

Equilibrium water absorption is determined in accordance with the following test method. The material to be tested is cut into 25.4 mm x 25.4 mm (1 inch x 1 inch) test pieces (about 25.4 $\mu$m (1 mil) thick) and the test pieces are weighed. The test pieces are then immersed in water for 90 minutes at ambient conditions. The test pieces are then removed from the water, blotted gently to remove excess liquid and re-weighed. The percent weight gain is calculated for each test piece. The test result is reported as the average of 2 determinations.

Dry tensile strength is determined on a standard Instron tester using a one inch jaw gap and a crosshead speed of 5 inches per minute. The tests are run on test specimens having a length of 127 mm (5 inches), a width of 25.4 mm (1 inch) and a thickness of about 25.4 $\mu$m (1 mil). Test results, in kPa (pounds/sq. inch), are reported as the average of 2 determinations.

Wet tensile strength is determined by soaking test specimens 127 mm (5 inches) long, 25.4 mm (1 inch) wide and about 25.4 $\mu$m (1 mil) thick in water for 90 minutes at room temperature. The test specimens are then removed from the water, blotted gently to remove excess liquid and then tested in the Instron tester as described above for dry samples.

The 70% aqueous iso-propyl alcohol absorption of a film is determined according to the following procedure. An aqueous solution of 70% by volume of iso-propanol and 30% by volume of water is prepared. The material to be tested is cut into 25.4 mm x 25.4 mm (1 inch x 1 inch) test pieces about 25.4 $\mu$m (1 mil) thick and the test pieces are weighed. The test pieces are then immersed in the aqueous iso-propanol solution for 90 minutes at ambient conditions. The test pieces are then removed from the solution, blotted gently to remove excess liquid and re-weighed. The percent weight gain is calculated for each test piece. The test result is reported as the average of two determinations.

Polymers which are suitable for use in the second major layer can be selected from the following: polyether urethanes, polyester urethanes, polyureas and their ionomeric analogs, polyether-amides, polyether ester-amides, polyesters and polyether-ester block copolymers, it being understood that any such polymer or blend of polymers, when formed into a film having a thickness of approximately 25.4 $\mu$m (1 mil), must meet the aforementioned properties.

The bicomponent films of the present invention have thicknesses ranging from 10 $\mu$m (0.0004 inch) to 254 $\mu$m (0.010 inch). The thicknesses of the first major layer and the second major layer can be easily ascertained by those skilled in the art and will depend on such factors as the end use to which the bicomponent film will be put, the alcohol resistance of the polymers comprising the second major layer, the desired degree of breathability, etc.. As an example, it will be evident that the thickness of the second major layer can be reduced if the polymer of the first major layer has a higher degree of alcohol resistance.

The invention will be more clearly understood by reference to the following detailed description and the accompanying drawings in which:

Fig. 1 is a perspective of the bicomponent polymeric film of the invention, with one corner of the film being turned upwardly to show the lower surface thereof;

Fig. 2 is a cross-section taken along line 2-2 of Fig. 1;

Fig. 3 is a diagrammatic perspective of an extruding apparatus which can be used to prepare bicomponent polymeric films of the present invention; and

Fig. 4 is an enlarged sectional view of a portion of the apparatus shown in Fig. 3.

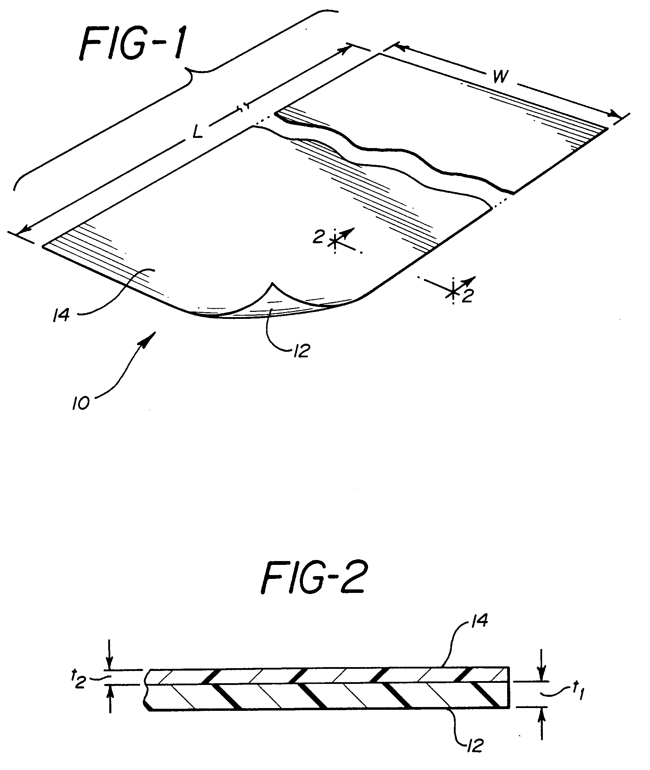

Referring now to the accompanying drawings, Fig. 1 shows a bicomponent polymeric film 10 of the present invention. It will be understood by those skilled in the art that film 10 may be provided in any desired combination of width, W, and length, L. As indicated earlier, film 10 comprises a first major layer 12 of polymeric material and a second major layer 14 of polymer material, with these major layers being joined together in face-to-face relationship throughout their length and width.

First major layer 12, which may be formed from any of the resin blends described in EP-A-0 476 963 mentioned earlier herein and recited in claim 1, comprises i) a hydrophilic block poly(ether-co-amide) containing between 20 and 80 weight percent polyethylene glycol (PEG) blocks and ii) a hydrophobic polymer.

The hydrophilic block poly(ether-co-amide) copolymer comprising first major layer 12 is a block copolymer with repeating "soft" polyether block and "hard" polyamide blocks. The soft polyether blocks enhance flexibility, hydrophilicity and thermoplasticity of the copolymer. The hard polyamide blocks enhance the tensile strength properties of the copolymer. The term "hydrophilic" as used to describe the poly(ether-co-amide) refers to the ability of such block copolymers to swell in water and to absorb at least 50 percent by weight of water at equilibrium.

The polyether blocks of the hydrophilic copolymer contain blocks of polyethylene glycol (PEG), with the amount of PEG blocks in the copolymer ranging from 20 to 80 percent by weight of the copolymer. When the amount of PEG in the block copolymer is less than 20 percent by weight, the bicomponent film 10 of the invention may not have an acceptable MVTR. If the amount of PEG in the copolymer exceeds 80 percent by weight, the dimensional integrity of the bicomponent film may be compromised.

In addition to PEG, the polyether blocks of the copolymer may also contain separate, repeating units of other polyalkylene oxides, such as propylene oxide, provided that the total amount of PEG in the copolymer is between 20 percent and 80 percent by weight. In a similar fashion, PEG can be copolymerized with other alkylene oxides to form suitable polyether blocks.

Preferably, the polyether blocks of the hydrophilic block copolymer consist only of repeating units of PEG and the number average molecular weight of each of the PEG blocks ranges from 600 to 8000. Preferably, the molecular weight of the PEG blocks ranges from 800 to 3000.

The polyamide blocks of the hydrophilic block copolymer may be polyamides such as nylon 6, 6-6, 6-12, 11 or 12. In addition, the polyamide blocks may contain repeating units of dissimilar polyamides, or copolyamides prepared by reacting at least two different polyamides.

The molecular weight of the hydrophilic block copolymer of the first major layer should be sufficiently high to provide, in conjunction with the polymeric component(s) used in the second major layer, a bicomponent film having acceptable dimensional stability for its intended end use. As a general rule, the weight average molecular weight, $M_w$, of the hydrophilic block copolymer, as determined by gel permeation chromatography, should be greater than about 20,000.

Hydrophilic block copolymers suitable for inclusion in first major layer 12 are known and can be obtained on a commercial basis from several sources. In one suitable copolymer, polyether blocks are linked to polyamide blocks through ester bonds. Such copolymers are described generally in U.S. 4,252,920, U.S. 4,115,475 and U.S. 4,208,493. In another suitable copolymer, polyether blocks are terminated with amine groups and linked directly to polyamide blocks, as described in U.S. 4,808,675. The most preferred hydrophilic block copolymer for inclusion in first major layer 12 is a poly(ether-co-amide) supplied by Atochem of North America as PEBAX™ MX1657.

The amount of hydrophilic block poly(ether-co-amide) copolymer required in first major layer 12 of bicomponent film 10 will depend on the desired balance between MVTR and wet strength. The specific amount will be easily selected by those skilled in the art. The amount of the hydrophilic block copolymer in the first major layer desirably ranges from about 20 to about 90 percent by weight, and preferably from about 30 to about 70 percent by weight, of the polymeric film-forming components comprising the first major layer.

The first major layer of polymeric material further comprises a hydrophobic polymer component which, in a manner similar to the aforementioned hydrophilic block poly(ether-co-amide), should have a weight average molecular weight greater than about 20,000 so as to be suitable as a film forming component of the first major layer. The term "hydrophobic", when used to describe a hydrophobic polymer to be included in the first major layer, refers to a polymer whose equilibrium water absorption, as measured by the test method described earlier herein, is less than about 10 weight percent.

One of the hydrophobic polymers which can be included in the first major layer is a block poly(ether-co-amide) containing essentially no PEG blocks. The term "essentially no PEG blocks" is intended to encompass block poly(ether-co-amide) polymers which contain a minimal amount of PEG blocks provided that the presence of said PEG blocks do not increase the equilibrium water absorption of the block copolymer to an amount greater than about 10 percent by weight. Preferably, the hydrophobic block copolymer contains no PEG blocks and contains polyether blocks of polytetramethylene oxide or polypropylene oxide. Such hydrophobic block copolymers are known and described in U.S. 4,252,920. Preferred hydrophobic polymers for inclusion in the first major layer are the block poly(ether-co-amide) copolymers commercially available from Atochem, North America under the tradenames PEBAX™ 2533, 4033 and 3533. These are block copolymers of poly(tetramethylene oxide) and nylon-12 connected with ester linkages. For example, PEBAX™ 4033 is a block copolymer of poly(tetramethylene oxide) and nylon-12, wherein the poly(tetramethylene blocks) have a molecular weight of about 2000 and the polyamide blocks have a molecular weight of about 2000. This polymer has good tensile strength and elongation properties, good abrasion resistance, low water absorption and moderate gas permeability.

Other hydrophobic polymers besides those mentioned in the preceding paragraph may be used in the first major layer. Such other polymers are polyesters, polyamides and polyurethanes. Polyesters which can be used are conventionally prepared by reacting a poly(alkylene oxide) with a dicarboxylic acid, and are described, for example, in U.S. 4,725,481. Particularly preferred polyesters include polycaprolactone and poly(tetramethylene glycol) terephthalate. Polycaprolactone is a particularly preferred polyester for use as the hydrophobic polymer in the first major layer. Examples of polyamides which can be utilized as the hydrophobic polymer in the first major layer include nylon 6, 6-6, 11 and

4

12, as well as copolyamides prepared by copolymerizing two or more of these. Suitable polyurethanes which can be included in the first major layer are conventional hydrophobic polyether urethanes or polyester urethanes. Generally, these polyurethanes are prepared by reacting excess diisocyanate with a hydroxyl-terminated polyether or polyester which can be chain extended with low molecular weight diols or diamines. Diisocyanates which may be used in the preparation of the mentioned polyurethanes include toluene diisocyanate (TDI); 4,4-diphenylmethane diisocyanate (MDI) and hexamethylene diisocyanate. Polyether polyols which may be used in the preparation of the mentioned polyurethanes include polypropylene glycol and polytetramethylene glycol. Examples of polyester polyols which may be used in the preparation of the mentioned polyurethanes include ethylene glycol adipate, butanediol succinate and neopentyl glycol azelate.

The preferred hydrophobic polymers for inclusion in the first major layer are the block poly(ether-co-amides) containing essentially no PEG blocks, polyamides and polyurethanes. The most preferred hydrophobic polymers are the block poly(ether-co-amides) and the polyamides.

Additional polymeric components can be included in the first major layer without departing from the scope of the invention. Polyolefins, e.g. polyethylene or ionomer resins, such as Surlyn™ ionomer resin, can be incorporated into the first major layer. A highly viscous polyolefin, such as a linear low density polyethylene with a melt index less than 3, can be incorporated into the first major layer in amounts up to 30 percent by weight of the total film-forming polymeric components to enhance the processing characteristics. Ionomer resins can be added to increase the compatibility of the polymer components. Additionally, as is well known, additives such as antioxidants, lubricants, pigments, etc. can be included in the first major layer.

An additional polymeric component which can be included in the first major layer is a grafted polyolefin prepared by reacting a polyolefin with an alpha-beta ethylenically unsaturated polycarboxylic acid or anhydride Examples of polyolefins are polyethylene, polypropylene, ethylene-propylene rubber and the like. Examples of alpha-beta ethylenically unsaturated polycarboxylic acids or anhydrides are maleic anhydride, maleic acid and fumaric acid. Such grafted polyolefins can be incorporated into the first major layer in amounts up to 40 weight percent, preferably between about 5 and about 25 weight percent. Incorporation of a grafted polyolefin into the first major layer can significantly improve the compatibility of the polymeric film-forming components and enhance processing characteristics.

The second major layer 14 of the bicomponent film 10 of the present invention comprises a polymer or a mixture of two or more polymers which meet the criteria set forth below.

As indicated earlier herein, the polymer or mixture of polymers comprising the second major layer must be selected so that a film having a thickness of 25.4 μm (1 mil) prepared therefrom has the following properties:

a) an MVTR of greater than 1,000 g/sq.m/day of water as measured by the gravimetric test method set forth in ASTM E-96 (Dessicant Method);
b) an equilibrium water absorption of less than 25% by weight as determined by the previously described equilibrium water absorption test method; and
c) a 70% aqueous iso-propyl alcohol absorption of less than 50% by weight as determined by the previously described 70% aqueous iso-propyl alcohol absorption test method.

Polymers which are suitable for use in the second major layer of the bicomponent film of the present invention are polyether urethanes, polyester urethanes, polyureas and their ionomer analogs; polyether ester-amides; polyesters and polyether ester block copolymers.

Suitable polyether ester-amides are those supplied by Atochem of North America under the tradename PEBAX™.

These block copolymers generally comprise hydroxyl-terminated polyoxyethylene, polyoxypropylene or polyoxytetramethylene oligomers having a molecular weight of from about 500 to about 3000 linked by polyamide blocks.

The poly(ether-ester) copolymers are supplied commercially by E.I. duPont under the tradename Hytrel™. These block copolymers generally comprise hydroxyl terminated polyoxyethylene, polyoxypropylene or polyoxytetramethylene oligomers having a molecular weight of from about 400 to about 3500 linked by polyester blocks.

Certain commercially available polyurethanes made by reacting diisocyanates with hydroxyl-terminated polyesters or polyethers are also suitable for inclusion in the second major layer. Such polymers are commercially available, for example, from B.G. Goodrich Company under the tradename Estane™ and Dow Chemical Company under the tradename Pellethane™. Specific Estane™ polymers which are suitable for use in the second major layer include Estane 58206, 5714 and 58866. A Pellethane™ polymer which is suitable for use in the second major layer is Pellethane™ 2103-70A.

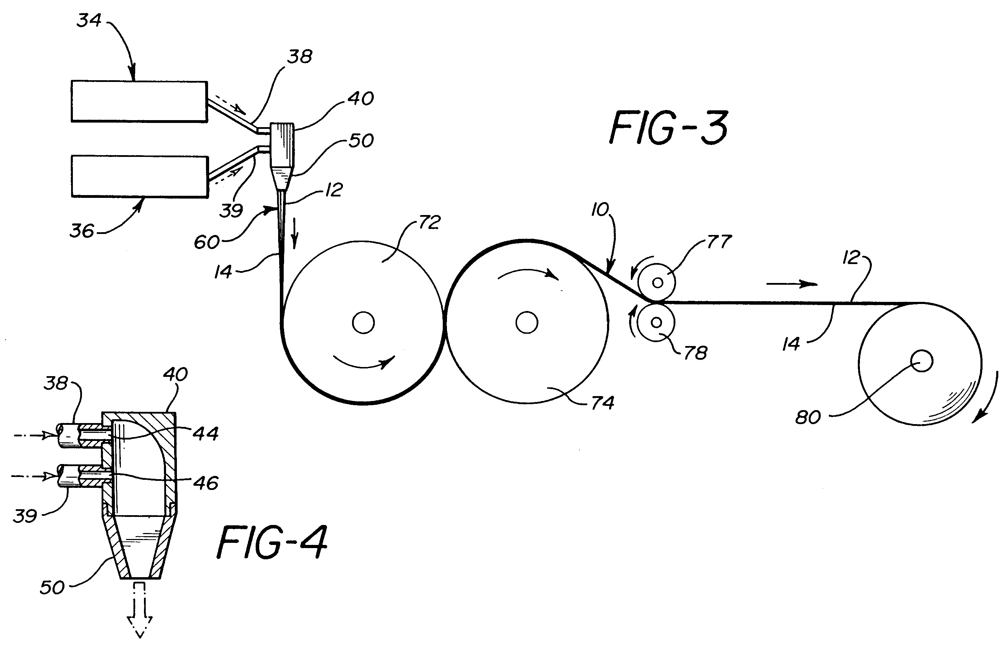

Bilayer films in accordance with the present invention are most conveniently made by co-extrusion, a process well known in the art of film manufacture. Referring to Fig. 3, there is schematically illustrated an apparatus and process for preparing the bilayer film of the present invention. The co-extrusion apparatus comprises a first extruder 34; a second extruder 36; a combining block 40; a single manifold flex lip die 50; a pair of driven chill rolls, 72 and 74, each suitably mounted for rotation; a pair of driven pull rolls, 77 and 78, each also mounted for rotation; and a rotating take-up roll 80.

Extruders 34 and 36 are 2.5 inch single screw extruders commercially available from Welex Co. under Model No. 2.50/30:1/V.L.H.. The extruder screw is 2.46 m (97 inches) in length and has a pitch of 30:1. Each extruder is equipped with a hopper (not shown in the drawings) from which material to be extruded is fed to the extruder inlet. The barrel of each extruder comprises 5 zones along its length, each of said zones being capable of being independently heated to a desired temperature.

Combining block 40, which is circular in cross-section, comprises a first inlet 44, a second inlet 46 and a single outlet connected directly to the inlet side of die 50.

The outlet of first extruder 34 is connected by suitable piping 38 to inlet 44 of combining block 40. The outlet of second extruder 36 is connected by suitable piping 39 to inlet 46 of combining block 40. The connecting piping is suitably heated so as to maintain the materials flowing therethrough in their molten state. A cooling liquid, typically held at 16°C (60°F), is continuously circulated through chill rolls 72, 74 during the co-extrusion process. Combining block 40 and die 50 are maintained, e.g., by electrical heating elements, at a temperature sufficiently high to maintain the materials passing therethrough in a molten state.

The polymers and additives, if any, comprising first major layer 12 of bicomponent film 10 are fed in a molten state by first extruder 34 to inlet 44 of combining block 40. The polymer(s) and additives, if any, comprising second major layer 14 of bicomponent film 10 are fed in a molten state by second extruder 36 to inlet 46 of combining block 40. It will be understood that the thickness of the first and second major layers of the film of the invention is controlled by the feed rates from first extruder 34 and second extruder 36, respectively. The components comprising the first major layer of the film are brought into contact with the components comprising the second major layer of the film as the molten materials flow through combining block 40 and die 50. The molten co-extrudate 60 drops a short distance, e.g., about 25.4 mm (1 inch), onto rotating chill roll 72 as illustrated in Fig. 3 of the accompanying drawings. The extrudate is led about one-half way around the outer surface of chill roll 72 where it is led onto the outer surface of chill roll 74. The bicomponent film 10 is drawn off chill roll 74, in the manner illustrated in Fig. 3, by a pair of pull rolls 77 and 78. The bicomponent film 10 is then wound up on a take-up roll 80.

## Example 1

A bicomponent polymeric film in accordance with the teachings of the present invention was made as follows. The first major layer of the bicomponent film had the following composition:

| Component | Description | Parts by Weight |
|---|---|---|
| PEBAX™ MX1657 | Polyether-ester Block Copolymer | 64.34 |
| Ultramid™ B35 | Nylon-6 | 14.84 |
| Fusabond MB-110 D | Polyethylene-Maleic Anhydride Graft Copolymer | 19.80 |
| Irganox™ 1035 | Antioxidant | 0.32 |
| Irganox™ MD 1024 | Antioxidant | 0.32 |
| Irgafos™ 168 | Heat Stabilizer | 0.23 |
| Chimasorb™ 944FL | UV Light Stabilizer | 0.15 |
| | | 100.00 |

PEBAX™ MX1657 is PEBAX™ 4011 having uniformly distributed therein 500 ppm Irganox™ 1010 (a heat stabilizer) and was obtained from Atochem of America, Birdsboro, PA. Ultramid™ B35 was obtained from BASF, Parsippany, NJ. Fusabond™ MB-110 D was obtained from E. I. duPont, Wilmington, DE. Irganox™ 1035 and MD 1024 and Irgafos™ 168 and Chimasorb™ 944FL were obtained from Ciba-Geigy, Hawthorne, NY.

The film forming polymers and additives comprising the first major layer were converted to pellets prior to their introduction into first extruder 34. The materials, except for the PEBAX™ MX1657, were dry-blended until uniform and were then fed to the inlet of a Werner & Pfleiderer ZSK-30 twin-screw extruder having a 44:1 length to diameter ratio. The ZSK-30 extruder has provision for introducing polymer pellets downstream of the inlet thereof, i.e. at a point approximately half way down the length of its barrel. In addition, the screw design of this particular extruder provides very good mixing and shearing characteristics. The extruder comprises a feed throat at the rear thereof and has eight zones whose temperatures can be set as desired. The PEBAX™ MX1657 polymer was added to the extruder at the aeremen-

tioned downstream addition point. The extruder was operated at 500 RPM with a throughput of 25 kg (55 pounds) per hour. The last seven Zones of the extruder barrel were set at a temperature of 240°C. The feed throat was continuously cooled, and the first heated zone (located adjacent the feed throat) was maintained at 100°C. The materials comprising the first major layer were thoroughly mixed as they passed through the extruder. The materials exited the extruder in the form of strands which were passed through a cold water quench tank and subsequently chopped into pellets (Mixture A pellets). The Mixture A pellets were dried and stored in sealed drums for subsequent use.

The second major layer of the bicomponent film consisted of PEBAX™ 4033. A 25.4 $\mu$m (1 mil) film (dry) of PEBAX™ 4033 was prepared by a conventional extrusion technique. The 25.4 $\mu$m (1 mil) film had a MVTR of 1427 g/sq.m/day ; an equilibrium water absorption of 0.8%; an equilibrium 70% aqueous isopropyl alcohol absorption of 17.3%; a MD dry tensile strength of 32.7 MN/m$^2$ (4743 pounds per square inch (psi)), a MD wet tensile strength (i.e. tensile strength of the film at its equilibrium water content) of 28.2 MN/m$^2$ (4087 psi), and a wet tensile strength retention of 86%. A 12 $\mu$m (0.48 mil) film of PEBAX™ 4033 had an MVTR of 2327 g/sq.m/day.

The bicomponent film of this Example 1 was made as follows. The aforementioned Mixture A pellets were charged to the hopper of first extruder 34 which was set to operate at 55 rpm. The five heating zones of the extruder barrel were set at the following temperatures:

Zone 1 (nearest the extruder inlet): 196°C (385°F);
Zone 2: 204°C (400°F); Zone 3: 216°C (420°F); Zone 4: 221°C (430°F); and
Zone 5 (nearest the extruder outlet): 221°C (430°F).

The connecting piping 38, feed block 40 and die 50 were maintained at a temperature of 221°C (430°F) during the extrusion process.

The Mixture B pellets, i.e., the PEBAX™ 4033 pellets, were charged to the hopper of second extruder 36 which was set to operate at 15 rpm. The extruder barrel zones and connecting piping associated with the second extruder were maintained at the same temperatures as the corresponding portions of the first extruder. The Mixture A pellets and Mixture B pellets were heated to a molten state in the first and second extruders, respectively. The Mixture A pellets in their molten state were brought into contact with the Mixture B pellets in their molten state in combining block 40 and then exited die 50 as a two-layered co-extrudate 60 comprising a first major layer 12 having the composition of the Mixture A pellets and a second major layer 14 having the composition of the Mixture B pellets. Co-extrudate 60 exited die 50 at a rate of about 3.7 m (12 feet) per minute. As co-extrudate 60 exited die 50, it had an approximate thickness of 0.38 mm (0.015 inch), of which the first major layer had a thickness of 0.30 mm (0.012) inch and the second major layer a thickness of 0.08 mm (0.003) inch. Owing to the fact that chill roll 72 was operated at a speed of 67 m/min (220 fpm), the thickness of co-extrudate 60 was decreased to a thickness of about 20 $\mu$m (0.0008 inch) by the time it came into contact with chill roll 72. Of this thickness,, about 16 $\mu$m (0.00064 inch) was contributed by the first major layer and about 4 $\mu$m (0.00016 inch) was contributed by the second major layer. Co-extrudate 60 was cooled to its final film form by the time it was taken off chill roll 72 and led onto chill roll 74. The resulting nonporous, breathable polymeric bicomponent film 10 was then rolled up on take-up roll 80.

The bicomponent film of Example 1 was tested and found to have the following properties:

| | |
|---|---|
| MVTR (g/sq.m/day) (First major layer exposed to the high humidity source during testing) | 2688 |
| Tensile Strength | |
| • Machine Direction | 48.4 MN/m$^2$ (7025 psi) |
| % Elongation to Break | 580 |
| Thickness in $\mu$m (inches) | |
| • Film | 20 (0.0008) |
| • First Major Layer, $t_1$ | 16 (0.00064) |
| • Second Major Layer, $t_2$ | 4 (0.00016) |
| • Ratio, $t_1$:$t_2$ | 4:1 |
| Colored Alcohol Solution Strikethrough Test | pass |

The Colored Alcohol Solution Strikethrough Test was conducted in accordance with the following procedure:

Colored Alcohol Solution Strikethrough Test

Purpose:

The purpose of this test is to demonstrate the effectiveness of the second major layer in preventing an aqueous isopropanol solution from coming into contact with the first major layer of the bicomponent film.

General Description:

A 70:30 volume percent isopropanol:water solution with 0.33% by weight FD&C Red Dye #4 is used to determine whether the second major layer protects the first major layer from the alcohol solution by observing whether or not the colored alcohol solution diffuses through the layers and into a white pulp board support.

Test Method:

1) Prepare the colored alcohol solution as follows: Dissolve 0.26 grams of FD&C Red Dye #4 in 30 ml. of water then add 70 ml. of isopropyl alcohol and mix thoroughly.

2) Secure a minimum of 0.093 $m^2$ (1 square foot) of the film to be tested to white pulp board with the second major layer facing upwardly.

3) Place a 500 gram weight with a 5 cm x 3.2 cm (2" x 1 1/4") flat surface on top of two 10.2 cm x 10.2 cm (4" x 4") RAYTEC™ Sponges.

4) Using 5 ml. of the mentioned colored alcohol solution per square foot of film, pour the solution down the middle of the film sample.

5) Promptly spread the solution uniformly over the film with the weighted sponges. This spreading process should be done within 15 seconds. The sponges and weight are then removed from the specimen.

6) Allow the solution to dry completely.

7) Remove the film and inspect the pulp board for red color. If there is no red color on the pulp board then the sample passes the test.

A control film, consisting solely of the components of the first major layer, was prepared by extrusion. The control film had a thickness of 18 $\mu$m (0.0007 inch) and had poor resistance to 70% aqueous isopropanol solution. The control film was tested and found to have the following properties:

| MVTR (g/sq.m/day) | 4548 |
|---|---|
| Tensile Strength | |
| • Machine Direction | 34.6 MN/m$^2$ (5021 psi) |
| % Elongation to Break | 458 |

Example 2

Another bicomponent film in accordance with the present invention was prepared. The first major layer of the bicomponent film was made from the same Mixture A pellets used in Example 1.

The second major layer of the film of this Example 2 was made from Mixture B pellets which consisted of a blend of 80 pbw PEBAX™ 4033 and 20 pbw of PEBAX™ 4011. A 23 $\mu$m (0.9 mil) thick film was extruded from the Mixture B pellets. This film was tested and found to have the following properties: MVTR of 2210 g/sq.m/day (equivalent to 1989 g/sq.m/day at 25.4 $\mu$m (1 mil) of film thickness); an equilibrium water absorption of 20.9%; an equilibrium 70% aqueous isopropyl alcohol absorption of 39%; a MD dry tensile strength of 34.5 MN/m$^2$ (5000 pounds per square inch), a MD wet tensile strength (i.e. tensile strength of the film at its equilibrium water content) of 33.4 MN/m$^2$ (4850 psi), and a wet tensile strength retention of 97%.

The apparatus used was the same as that used in Example 1. The Mixture A pellets were charged to the hopper of first extruder 34 and the Mixture B pellets were charged to the hopper of second extruder 36. The extruder barrel zones of both extruders, connecting pipes 38 and 39, combining block 40 and die 50 were set at the same temperatures as those used in Example 1. First extruder 34 was operated at 35 rpm; second extruder 36 was operated at 35 rpm. Chill rolls 72, 74, take-off rolls 77, 78 and wind-up roll 80 operated at 76 m/min (250 feet per minute (fpm)). Co-extrudate 60 exited die 50 at a rate of about 4.6 m/min (15 fpm). As co-extrudate 60 exited die 50, it had an approximate thickness of 0.38 mm (0.015 inch), of which 0.19 mm (0.0075 inch) was contributed by the first major layer and about 0.19 mm (0.0075 inch) was contributed by the second major layer. Owing to the fact that chill roll 72 was operated at 76 m/min (250 fpm), the thickness of co-extrudate 60 was decreased to about 23 $\mu$m (0.0009 inch) by the time it came into contact with roll 72. Of this thickness, about 11.4 $\mu$m (0.00045 inch) was contributed by the first major layer and about 11.4 $\mu$m (0.00045 inch) was contributed by the second major layer. As in Example 1, co-extrudate 60 was solidified to its final film form by the time it was taken off chill roll 72 and led onto chill roll 74. The resulting nonporous, breathable polymeric bicomponent film 10 of this Example 2 was wound up onto roll 80.

The film of this Example 2 was tested and found to have the following characteristics.

| MVTR (g/sq.m/day) (First major layer exposed to the high humidity source during testing) | 3438 |
|---|---|
| Tensile Strength | |
| • Machine Direction | 54.0 MN/m$^2$ (7838 psi) |
| % Elongation to Break | 258 |
| Thickness in $\mu$m (inches) | |
| • Film | 22.8 (0.0009) |
| • First Major Layer, $t_1$ | 11.4 (0.00045) |
| • Second Major Layer, $t_2$ | 11.4 (0.00045) |
| • Ratio, $t_1$:$t_2$ | 1:1 |
| Colored Alcohol Solution Strikethrough Test | pass |

Example 3

Another bicomponent film in accordance with the present invention was prepared. The first major layer of the bicomponent film was made from the same Mixture A pellets used in Example 1.

The second major layer of the film of this Example 3 was made from Mixture B pellets which consisted of Pellethane™ 2103-70A, a polyether urethane copolymer commercially available from Dow Chemical, Midland, Michigan.

A 1.1 mil film (dry) of Pellethane™ 2103-70A was prepared by extrusion. The 28 $\mu$m (1.1 mil) film had an MVTR of 1264 g/sq.m/day (equivalent to 1390 g/sq.m/day at 25.4 $\mu$m (1 mil) film thickness); an equilibrium water absorption of 21%; an equilibrium 70% aqueous isopropyl alcohol absorption of 38%; a dry tensile strength of 51.9 MN/m$^2$ (7522 pounds per square inch), a wet tensile strength (i.e. tensile strength of the film at its equilibrium water absorption) of 38.4 MN/m$^2$ (5575 psi), and a wet tensile strength retention of 76%.

The apparatus used was the same as that used in Example 1 except that combining block 40 was not used and die 50 was replaced by a dual manifold slot die; pull rolls 77, 78 were not used; and extruders 34, 36 were 25.4 mm (1.0 inch), single screw extruders commercially available from Killion Company under Model No. KL100. The extruder screws were 0.61 m (24 inches) long and had a pitch of 24:1. The barrels of each of the extruders had three heatable zones. The Mixture A pellets were charged to the hopper of first extruder 34 and the Mixture B pellets were charged to the hopper of second extruder 36. The three heatable zones of extruder 34 were set at the following temperatures:

Zone 1 (nearest the extruder inlet): 196°C (385°F);
Zone 2: 216°C (420°F);
Zone 3: (nearest the extruder outlet): 221°C (430°F);

The three heatable zones of extruder 36 were held at the following temperatures:

Zone 1 (nearest the extruder inlet): 188°C (370°F);
Zone 2: 196°C (385°F);
Zone 3: 204°C (400°F);

Connecting pipe 38 was maintained at 221°C (430°F) and connecting pipe 39 was maintained at 204°C (400°F). The slot die was held at 221°C (430°F). First extruder 34 was operated at 50 rpm; second extruder 36 was operated at 20 rpm. Chill rolls 72, 74, and wind-up roll 80 operated at 14.6 m/min (48 feet per minute (fpm)).

Co-extrudate 60 exited the die at a rate of about 1.5 m/min (4.8 fpm). As co-extrudate 60 exited the die, it had an approximate thickness of 0.25 mm (0.010 inch), of which 0.18 mm (0.0071 inch) was contributed by the first major layer and about 0.07 mm (0.0029 inch) was contributed by the second major layer. Owing to the fact that chill roll 72 was operated at 14.6 m/min (48 fpm), the thickness of co-extrudate 60 was decreased to about 25.4 $\mu$m (0.001 inch) by the time it came into contact with roll 72. Of this thickness, about 18.0 $\mu$m (0.00071 inch) was contributed by the first major layer and about 7.4 $\mu$m (0.00029 inch) was contributed by the second major layer. As in Example 1, co-extrudate 60 was solidified to its final film form by the time it was taken off chill roll 72 and led onto chill roll 74. The resulting nonporous, breathable polymeric bicomponent film 10 of this Example 3 was wound up onto roll 80.

The film of this Example 3 was tested and found to have the following characteristics.

| | |
|---|---|
| MVTR (g/sq.m/day) (First major layer exposed to the high humidity source during testing) | 2600 |
| Tensile Strength | |
| • Machine Direction | 23.2 MN/m$^2$ (3362 psi) |
| % Elongation to Break | 394 |
| Thickness in $\mu$m (inches) | |
| • Film | 25.4 (0.001) |
| • First Major Layer, $t_1$ | 18.0 (0.00071) |
| • Second Major Layer, $t_2$ | 7.4 (0.00029) |
| • Ratio, $t_1$:$t_2$ | 2.45:1 |
| Colored Alcohol Solution Strikethrough Test | pass |

Example 4

A fourth bicomponent film in accordance with the present invention was prepared. The first major layer of the bicomponent film was made from the same Mixture A pellets used in Example 1.

The second major layer of the film of this Example 4 was made from Mixture B pellets which consisted of Estane 5714, a polyester urethane copolymer commercially available from B. F. Goodrich.

A 33 $\mu$m (1.3 mil) film (dry) of Estane™ 5714 was prepared by a conventional extrusion technique. The 33 $\mu$m (1.3 mil) film had an MVTR of 1149 g/sq.m/day (equivalent to 1493 g/sq.m/day at 25.4 $\mu$m (1 mil) of film thickness); an equilibrium water absorption of 5.5%; an equilibrium 70% aqueous isopropyl alcohol absorption of 29%; a MD dry tensile strength of 63.6 MN/m$^2$ (9230 pounds per square inch); a wet tensile strength (i.e. tensile strength of the film at its equilibrium water content) of 23.2 MN/m$^2$ (3362 psi), and a wet tensile strength retention of 36%.

The apparatus used was the same as that used in Example 3. The Mixture A pellets were charged to the hopper of first extruder 34 and the Mixture B pellets were charged to the hopper of second extruder 36. The extruder barrel zones of both extruders, connecting pipes 38 and 39, and the die were held at the same temperatures as those used in Example 3, except zones 1 and 2 of second extruder 36 were held at temperatures of 182°C (360°F) and 193°C (380°F), respectively. First extruder 34 was operated at 50 rpm; second extruder 36 was operated at 20 rpm. Chill rolls 72, 74 and wind-up roll 80 operated at 17.4 m/min (57 feet per minute (fpm)). Co-extrudate 60 exited the die at a rate of about 1.74 m/min (5.7 fpm). As co-extrudate 60 exited the die, it had an approximate thickness of 0.254 mm (0.010 inch), of which 0.175 mm (0.0069 inch) was contributed by the first major layer and about 0.079 mm (0.0031 inch) was contributed by the second major layer. Owing to the fact that chill roll 72 was operated at 17.4 m/min (57 fpm), the thickness of co-extrudate 60 was decreased to about 25.4 $\mu$m (0.001 inch) by the time it came into contact with roll 72. Of this thickness, about 17.5 $\mu$m (0.00069 inch) was contributed by the first major layer and about 7.9 $\mu$m (0.00031 inch) was contributed by the second major layer. As in Example 3, co-extrudate 60 was solidified to its final film form by the time it was taken off chill roll 72 and led onto chill roll 74. The resulting nonporous, breathable polymeric bicomponent film 10 of this Example 4 was wound up onto roll 80.

The film of this Example 4 was tested and found to have the following characteristics.

| MVTR (g/sq.m/day) (First major layer exposed to the high humidity source during testing) | 2838 |
|---|---|
| Tensile Strength | |
| • Machine Direction | $26.7 \text{ MN/m}^2$ (3875 psi) |
| % Elongation to Break | 285 |
| Thickness in $\mu$m (inches) | |
| • Film | 25.4 (0.001) |
| • First Major Layer, $t_1$ | 17.5 (0.00069) |
| • Second Major Layer, $t_2$ | 7.9 (0.00031) |
| • Ratio, $t_1$:$t_2$ | 2.2:1 |
| Colored Alcohol Solution Strikethrough Test | pass |

Example 5

A fifth bicomponent film in accordance with the present invention was prepared. The first major layer of the bicomponent film was made from the same Mixture A pellets used in Example 1.

The second major layer of the film of this Example 5 was made from Mixture B pellets which consisted of Hytrel™ G3548, a poly(ether-ester) copolymer commercially available from E. I. duPont.

A 25.4 $\mu$m (1 mil) film (dry) of Hytrel™ G3548 was prepared by a conventional extrusion technique. The 25.4 $\mu$m (1 mil) film had an MVTR of 2360 g/sq.m/day; an equilibrium water absorption of 0.4%; an equilibrium 70% aqueous iso-propyl alcohol absorption of 44.1%; a MD dry tensile strength of 9.63 MN/m$^2$ (1396 pounds per square inch (psi)), and a MD wet tensile strength (i.e. tensile strength of the film at its equilibrium water content) of 9.78 MN/m$^2$ (1419 psi). A 15 $\mu$m (0.59 mil) thick film of Hytrel™ G3548 had an MVTR of 3559 g/sq.m/day.

The apparatus used was the same as that used in Example 3. The Mixture A pellets were charged to the hopper of first extruder 34 and the Mixture B pellets were charged to the hopper of second extruder 36. The extruder barrel zones of both extruders, connecting pipes 38 and 39, and the die were held at the same temperatures as those used in Example 3, except zones 1 and 2 of second extruder 36 were held at temperatures of 193°C (380°F) and 213°C (415°F), respectively. First extruder 34 was operated at 50 rpm; second extruder 36 was operated at 20 rpm. Chill rolls 72, 74 and wind-up roll 80 operated at 15.5 m/min (51 feet per minute (fpm)). Co-extrudate 60 exited the die at a rate of about 1.55 m/min (5.1 fpm). As co-extrudate 60 exited the die, it had an approximate thickness of 0.254 mm (0.010 inch), of which 0.175 mm (0.0069 inch) was contributed by the first major layer and about 0.079 mm (0.0031 inch) was contributed by the second major layer. Owing to the fact that chill roll 72 was operated at 15.5 m/min (51 fpm), the thickness of co-extrudate 60 was decreased to about 25.4 $\mu$m (0.001 inch) by the time it came into contact with roll 72. Of this thickness, about 17.5 $\mu$m (0.00069 inch) was contributed by the first major layer and about 7.9 $\mu$m (0.00031 inch) was contributed by the second major layer. As in the other Examples, co-extrudate 60 was solidified to its final film form by the time it was taken off chill roll 72 and led onto chill roll 74. The resulting nonporous, breathable polymeric bicomponent film 10 of this Example 5 was wound up onto roll 80.

The film of this Example 5 was tested and found to have the following characteristics.

| MVTR (g/sq.m/day) (First major layer exposed to the high humidity source during testing) | 3156 |
|---|---|
| Tensile Strength | |
| • Machine Direction | 30.1 MN/m$^2$ (4368 psi) |
| % Elongation to Break | 330 |
| Thickness in $\mu$m (inches) | |
| • Film | 25.4 (0.001) |
| • First Major Layer, $t_1$ | 17.5 (0.00069) |
| • Second Major Layer, $t_2$ | 7.9 (0.00031) |
| • Ratio, $t_1$:$t_2$ | 2.2:1 |
| Colored Alcohol Solution Strikethrough Test | pass |

Films according to the present invention can be readily laminated to woven, knitted or nonwoven fabric substrates to provide breathable fabric/film laminates. Such laminates can be used in a variety of applications. For example, the laminates can be used to make surgical gowns worn by surgeons and other personnel in the operating room. The fabric/film laminates may be used to manufacture protective garments such as pants, jackets, head coverings, foot coverings and the like. Such protective garments would be worn by personnel working in areas where there are undesirable fumes, particulate matter, etc.. It will be recognized that fabric/film/fabric laminates, i.e. structures in which a layer of film according to the present invention is sandwiched between two outer layers of fabric, may also be readily made and usefully employed in the above-mentioned surgical gowns and protective garments.

Example 6

A fabric/film/fabric laminate employing a bicomponent film having the composition of the film of Example 1 was prepared as follows. A 17.0 g/m$^2$ (0.5 ounce/square yard), point embossed, nylon spunbonded nonwoven fabric was used as one of the fabric layers. This nonwoven fabric was obtained from Fiberweb, Inc. under the tradename Cerex™ Type 30.

The other fabric used to make the laminate of this Example 6 was a 33.9 g/m$^2$ (1.0 ounce/square yard), hydroentangled polyester fiber nonwoven fabric treated with a resin binder containing a flame retardant salt, a fluorochemical and pigment. The hydroentangled polyester fiber nonwoven fabric was made by first forming a web of 0.17 g/km (1.5 denier), 38 mm (1.5 inch) staple polyester fibers. The web was formed by a conventional carding technique and weighed 32.2 g/m$^2$ (0.95 ounce/square yard). The web was passed through a hydroentangling apparatus of the type disclosed in U.S. 3,485,706. In the apparatus, water jets were emitted from a series of rows of orifices having a diameter of about 0.127 mm (0.005 inch). The web to be hydroentangled was supported on a 100 x 92 bronze wire, twill weave belt (Appleton Wire Co. of Appleton, Wisconsin) as it was passed under the water jets. There were 12 orifices per cm (30 orifices per inch) in each of the rows. The water jets delivered 0.75 kW (1 horsepower) of energy to 0.45 kg (1 pound) of web material passing beneath the water jets in one hour. After completion of the hydroentangling process, the web was passed over a vacuum slot to remove excess processing water and was dried. The hydroentangled web was then saturated with a finishing composition consisting of 82.88 parts by weight (pbw) of water; 5.53 pbw of Airflex 4500 (an ethylene-vinyl chloride emulsion polymer); 5.63 pbw of Flameproof 736 (a flame retardant salt); and 5.05 pbw of Milease F31X (a fluorochemical repellent). The finishing bath also contained minor amounts of pigment to provide a green coloration as well as 0.07 pbw Hodag NC 24, a silicone antifoam. The saturated hydroentangled web was dried in the normal manner to provide the final hydroentangled nonwoven fabric. The fabric comprised about 5% by weight of the finish based on total fabric weight.

The first major layer and the second major layer of the bicomponent film used to prepare the fabric/film/fabric laminate of this Example 6 had the same compositions as the first major layer and the second major layer of the bicomponent film of Example 1.

The first major layer of the bicomponent film had a thickness of approximately 14 $\mu$m (0.00055 inch), while the second major layer of the bicomponent film had a thickness of approximately 5.1 $\mu$m (0.0002 inch).

The Cerex™ nonwoven fabric was laminated to the first major surface of the bicomponent film and the hydroentangled nonwoven fabric was laminated to the second major surface of the bicomponent film to provide the fabric/film/fabric

EP 0 560 630 B1

laminate of this Example 6. In the lamination process, a moisture curable polyurethane polymer dissolved in trichloroethylene was printed in a discontinuous pattern on one side of each of the aforementioned nonwoven fabrics. The adhesive coated surface of the Cerex™ nonwoven fabric was adhered to the first major surface of the bicomponent film and the adhesive coated surface of the hydroentangled polyester fiber nonwoven fabric was adhered to the second major surface of the bicomponent film. The resulting 3-ply laminate was held in fixed position until the solvent had evaporated and the polyurethane adhesive had cured. The amount of dry solids adhesive applied to each nonwoven fabric was 1.7 $g/m^2$ (0.05 ounce/square yard).

The resulting tri-component laminate was tested and found to have the properties shown in Table II.

Table II

| Basis Weight, $g/m^2$ (ounces/sq. yard) | 75 (2.2) |
|---|---|
| Grab Tensile Strength (ASTM D-5034) | |
| • Machine Direction | 18 kg (40 lbs). |
| • Cross Direction | 9.5 kg (21 lbs). |
| Elemendorf Tear Strength | |
| • Machine Direction | 1000 gms. |
| • Cross Direction | 2300 gms. |
| Hydrostatic Head[1] | >100 cms. |
| Flame Retardancy[2] | |
| • Machine Direction | 16 secs. |
| • Cross Direction | 20 secs. |
| Softness[3] | |
| • Machine Direction | 34 gms. |
| • Cross Direction | 14 gms. |
| MVTR (g/sq.m/day) (with the Cerex™ nonwoven fabric side of the laminate exposed to the high humidity source during testing) | 3200 |

1) AATCC Test No. 127-1974
2) NFPA Test No. 702
3) Handle-O-Meter

Example 7

A nonwoven/film/nonwoven laminate employing a bicomponent film having the composition of the bicomponent film of Example 1 was prepared as follows. A web of 0.33 g/km (3 denier) polyester core/polyethylene sheath fibers was prepared by a standard carding technique. The fibers were obtained from BASF Company under the designation BASF Type 1050 staple fiber. The fibers had a length of 38 mm (1 ½ inches). The web of sheath/core fibers was led onto an 80 x 80 mesh metal carrier belt and passed through a forced air oven at 149°C (300°F) to bond the fibers together, thus forming the first nonwoven fabric for use in this Example 7.

The second nonwoven fabric used to make the laminate of this Example 7 was a 33.9 $g/m^2$ (1.0 ounce/square yard), hydroentangled polyester fiber nonwoven fabric treated with a finishing composition containing a flame retardant salt, a fluorochemical and pigment. The hydroentangled polyester fiber nonwoven fabric was made by first forming a web of 0.17 g/km (1.5 denier), 38 mm (1.5 inch) staple polyester fibers. The web was formed by a conventional carding technique and weighed 47.5 $g/m^2$ (1.44 ounce/square yard). The web was passed through a hydroentangling apparatus of the type disclosed in U.S. 3,485,706. In the apparatus, water jets were emitted from a series of rows of orifices having a diameter of about 0.127 mm (0.005 inch). The web to be hydroentangled was supported on a 100 x 92 bronze wire, twill weave belt (Appleton Wire Co. of Appleton, Wisconsin) as it was passed under the water jets. Eleven rows of orifices were employed. There were 12 orifices per cm (30 orifices per inch) in each of the rows. The water jets delivered 0.75 kW (1 horsepower) of energy to 0.45 kg (1 pound) of web material passing beneath the water jets in one hour. After completion of the hydroentangling process, the web was passed over a vacuum slot to remove excess processing water and was dried. The hydroentangled web was then saturated with a finishing composition consisting of 88.26 parts by

13

weight (pbw) of water; 3.28 pbw of Airflex 4500 (an ethylene-vinyl chloride emulsion polymer having 50% solids); 3.06 pbw of Flameproof 736 (a flame retardant salt having 44% solids); and 3.01 pbw of Milease F31X (a fluorochemical repellent having 27% solids). The finishing bath also contained minor amounts of pigment to provide a green coloration as well as 0.07 pbw Hodag NC 24, a silicone antifoam. The saturated hydroentangled fabric was transferred to a pin tenter frame and stretched to 170% of its original width while the saturated hydroentangled fabric is overfed to the frame by 17%. The stretched fabric was passed through an oven, while maintaining it in its stretched state, to provide the stretched and finished hydroentangled nonwoven fabric. The fabric comprises about 5% by weight of treatment composition solids based on total fabric weight.

The first major layer and the second major layer of the bicomponent film used to prepare the fabric/film/fabric laminate of this Example 7 had the same compositions as the first major layer and the second major layer of the bicomponent film of Example 1.

The first major layer of the bicomponent film had a thickness of approximately 14.7 $\mu$m (0.00058 inch), while the second major layer of the bicomponent film had a thickness of approximately 5.6 $\mu$m (0.00022 inch).

The hot air bonded bicomponent fiber nonwoven fabric was laminated to the first major surface of the bicomponent film and the finished stretched hydroentangled nonwoven fabric was laminated to the second major surface of the bicomponent film to provide the nonwoven fabric/bicomponent film/nonwoven fabric of this Example 7. In the lamination process, a moisture curable polyurethane polymer dissolved in trichloroethylene was printed in a discontinuous pattern on each of the mentioned nonwoven fabrics which were then brought into contact with the bicomponent film (with their applied adhesive in contact with the film). The three components were held in place until the solvent evaporated and the polyurethane adhesive cured. The adhesive was applied to each of the nonwovens at a rate of 1.7 g/m$^2$ (0.05 ounce/square yard).

The resulting tri-component laminate was tested and found to have the properties shown in Table III.

Table III

| Basis Weight, g/m$^2$ (ounces/sq. yard) | 75 (2.2) |
|---|---|
| Grab Tensile Strength (ASTM D-5034) | |
| • Machine Direction | 15 kg (33 lbs.) |
| • Cross Direction | 9.5 kg (21 lbs.) |
| Hydrostatic Head[1] | >100 cms. |
| Flame Retardancy[2] | |
| • Machine Direction | >20 secs. |
| • Cross Direction | >20 secs. |
| Softness[3] | |
| • Machine Direction | 40 gms. |
| • Cross Direction | 30 gms. |
| MVTR (g/sq.m/day) (with hot air bonded nonwoven fabric side of laminate exposed to the high humidity source during testing) | 2700 |

1) AATCC Test No. 127-1974
2) NFPA Test No. 702 (Test not run beyond 20 seconds)
3) Handle-O-Meter

The tricomponent laminate of this Example 7 find a particular utility in the manufacture of surgical gowns used in a medical facility, i.e., an operating room. In manufacturing such gowns, the tricomponent laminate is oriented so that the first nonwoven fabric, i.e., the hot air bonded nonwoven fabric, which is joined to the first major layer of the bicomponent film, constitutes the inner surface of the gown and the second nonwoven fabric (i.e. the hydroentangled web of polyester fibers), which is joined to the second major layer of the bicomponent film, constitutes the outer surface of the gown. If any blood, saline solution, aqueous isopropyl alcohol solutions or the like coming into contact with the hydroentangled nonwoven fabric on the outer surface of the gown manages to penetrate through the thickness of that nonwoven fabric, it would then come into contact with the second major layer of the bicomponent film. This second major layer, which as seen herein is highly resistant to diffusion therethrough by aqueous isopropanol solutions, then functions to help prevent said isopropanol solution from compromising the integrity of the underlying first major layer of bicomponent film.

**Claims**

1. A nonporous, breathable bicomponent polymeric film comprising a first major layer of polymeric material and a second layer of polymeric material,

   said first layer of polymeric material comprising:

   a) a hydrophilic block poly(ether-co-amide) which contains between 20 and 80 percent by weight based on the weight of the block poly(ether-co-amide), of polyethylene glycol blocks, and
   b) a hydrophobic polymer selected from the group consisting of a block poly(ether-co-amide) containing essentially no PEG blocks, a polyamide a polyester and a polyurethane;
   said second layer of polymeric material comprising a polymer or mixture of polymers selected from polyether urethane, a polyester urethane, a polyurea or one of its ionomeric analogs, a polyether ester-amide, a polyester, a polyether-ester block copolymer or a mixture thereof, and wherein said polymer or mixture of polymers upon being converted to a film having a thickness of 25.4μm (1 mil), has a moisture vapor transmission rate of at least 1000 g/sq.m/day; an equilibrium water absorption of less than 25%; and a 70% aqueous isopropanol solution absorption of less than 50%.

2. The polymeric film of Claim 1 having a thickness which ranges from 10μm (0.0004 inch) to 254μm (0.01 inch).

3. The polymer film of Claim 1 or 2 wherein the polymeric component of said second major layer comprises a polyether ester-amide block copolymer.

4. The polymeric film of Claim 1 or 2 wherein the polymeric component of said second major layer comprises a polyether urethane.

5. The polymeric film of Claim 1 or 2 wherein the polymeric component of said second major layer comprises a polyester urethane.

6. The polymeric film of Claim 1 or 2 wherein the polymeric component of said second major layer comprises a poly(ether-ester) copolymer.

7. A laminated structure comprising a first fabric joined to one of the major surfaces of the bicomponent film of any one of Claims 1 to 6.

8. The laminated structure of Claim 7 wherein said fabric is a woven fabric, a knitted fabric or a nonwoven fabric.

9. The laminated structure of Claim 8 wherein said fabric is a nonwoven fabric.

10. The laminated structure of Claim 9 wherein said nonwoven fabric comprises polyamide fibers and is point bonded.

11. The laminated structure of claim 9 wherein said nonwoven fabric comprises a hydroentangled web of polyester fibers.

12. The laminated structure of Claims 9 to Claim 11 wherein said nonwoven fabric is fire-retardant and is secured to the second major surface of said bicomponent film.

13. The laminated structure of any one of Claims 7 to 12 wherein said first fabric is joined to said bicomponent film by a discontinuous layer of adhesive.

14. The laminated structure of Claim 13 wherein said adhesive is a moisture curable polyurethane adhesive.

15. The laminated structure of any of Claims 7 to 11 wherein said first fabric is joined to the first major surface of said bicomponent film.

16. A tricomponent laminate comprising a first nonwoven fabric, a second nonwoven fabric and the bicomponent film of any one of Claims 1 to 6, said first nonwoven fabric comprising a layer of hot air bonded synthetic fiber, said second nonwoven fabric being fire retardant and comprising a hydroentangled web of staple length fibers, said first nonwoven fabric being joined to the first major surface of said bicomponent film and said second nonwoven fabric being joined to the second major surface of said bicomponent film.

**Patentansprüche**

1. Ein porenfreier, atmungsfähiger, polymerer Bikomponentenfilm, der eine erste Hauptschicht polymeres Material und eine zweite Schicht polymeres Material umfaßt, wobei die erste Schicht polymeres Material umfaßt:

   a) ein hydrophiles Blockpoly(ether-co-amid), das zwischen 20 und 80 Gewichtsprozent, bezogen auf das Gewicht des Blockpoly(ether-co-amid), an Polyethylenglykol-Blöcken enthält und

   b) ein hydrophobes Polymer, das aus der Gruppe, die aus Blockpoly(ether-co-amid), welches im wesentlichen keine PEG-Blöcke enthält, einem Polyamid, einem Polyester und einem Polyurethan besteht, ausgewählt ist; wobei die zweite Schicht polymeres Material ein Polymer oder eine Mischung von Polymeren umfaßt, die aus Polyetherurethan, einem Polyesterurethan, einem Polyharnstoff oder einem seiner ionomeren Analoge, einem Polyetheresteramid, einem Polyester, einem Polyetherester-Block-Copolymer oder einer Mischung davon ausgewählt ist, und wobei das Polymer oder die Polymermischung nach Umwandlung in einen Film, der eine Dicke von 25,4 $\mu$m (1 mil) hat, eine Wasserdampfdurchlässigkeitsrate von mindestens 1000g/m$^2$ /Tag; eine Gleichgewichtswasserabsorption von weniger als 25 %; und eine Absorption von 70 %-iger wässriger Isopropanollösung von weniger als 50 % hat.

2. Der Polymerfilm nach Anspruch 1, mit einer Dicke, die zwischen 10 $\mu$m (0,0004 inch) und 254 $\mu$m (0,01 inch) liegt.

3. Der Polymerfilm nach Anspruch 1 oder 2, wobei die polymere Komponente der zweiten Hauptschicht ein Polyetheresteramid-Block-Copolymer umfaßt.

4. Der Polymerfilm nach Anspruch 1 oder 2, wobei die polymere Komponente der zweiten Hauptschicht ein Polyetherurethan umfaßt.

5. Der Polymerfilm nach Anspruch 1 oder 2, wobei die polymere Komponente der zweiten Hauptschicht ein Polyesterurethan umfaßt.

6. Der Polymerfilm nach Anspruch 1 oder 2, wobei die polymere Komponente der zweiten Hauptschicht ein Poly(ether-ester)-copolymer umfaßt.

7. Eine laminierte Struktur, die einen ersten Stoff umfaßt, welcher mit einer der Hauptoberflächen des Bikomponentenfilms nach einem der Ansprüche 1 bis 6 verbunden ist.

8. Die laminierte Struktur nach Anspruch 7, wobei der Stoff ein gewebter, gestrickter oder nicht-gewebter Stoff ist.

9. Die laminierte Struktur nach Anspruch 8, wobei der Stoff ein nicht-gewebter Stoff ist.

10. Die laminierte Struktur nach Anspruch 9, wobei der nicht-gewebte Stoff Polyamidfasern umfaßt und punktgebunden ist.

11. Die laminierte Struktur nach Anspruch 9, wobei der nicht-gewebte Stoff ein hydroverhaktes Netz von Polyesterfasern umfaßt.

12. Die laminierte Struktur nach Ansprüchen 9 bis 11, wobei der nicht-gewebte Stoff feuerhemmend und an der zweiten Hauptoberfläche des Bikomponentenfilms befestigt ist.

13. Die laminierte Struktur nach einem der Ansprüche 7 bis 12, wobei der erste Stoff mit dem Bikomponentenfilm durch eine diskontinuierliche Kleberschicht verbunden ist.

14. Die laminierte Struktur nach Anspruch 13, wobei der Kleber ein durch Feuchtigkeit aushärtbarer Polyurethankleber ist.

15. Die laminierte Struktur nach einem der Ansprüche 7 bis 11, wobei der erste Stoff mit der ersten Hauptoberfläche des besagten Bikomponentenfilms verbunden ist.

16. Ein Drei-Komponenten-Laminat, welches einen ersten nicht-gewebten Stoff, einen zweiten nicht-gewebten Stoff und den Bikomponentenfilm nach einem der Ansprüche 1 bis 6 umfaßt, wobei der erste nicht-gewebte Stoff eine Schicht aus heißluftgebundener synthetischer Faser umfaßt, der zweite nicht-gewebte Stoff schwer brennbar ist und ein hydroverhaktes Netz von Stapellängenfasern umfaßt, der erste nicht-gewebte Stoff mit der ersten Hauptoberfläche des Bikomponentenfilms verbunden ist, und der zweite nicht-gewebte Stoff mit der zweiten Hauptoberfläche des Bikomponentenfilms verbunden ist.

## Revendications

1. Film polymère bicomposant, non poreux, respirable comprenant une première couche principale de matériau polymère et une seconde couche de matériau polymère, ladite première couche de matériau polymère comprenant :

    a) un poly(éther-co-amide) séquencé hydrophile, qui contient entre 20 et 80 pour cent en poids par rapport au poids du poly(éther-co-amide) séquencé, de séquences de polyéthylèneglycol, et
    b) un polymère hydrophobe choisi dans l'ensemble constitué d'un poly(éther-co-amide) séquencé ne contenant pratiquement aucune séquence PEG, un polyamide, un polyester et un polyuréthane ; ladite seconde couche de matériau polymère comprenant un polymère ou un mélange de polymères choisi parmi un polyéther uréthane, un polyester uréthane, une polyurée ou l'un de ses analogues ionomères, un polyéther esteramide, un polyester, un copolymère séquencé polyétherester ou un mélange de ceux-ci, et dans lequel ledit polymère ou mélange de polymères, après avoir été converti en un film ayant une épaisseur de 25,4 $\mu$m (1 mil), a un taux de transmission de vapeur d'eau d'au moins 1 000 g /m$^2$/jour ; une absorption d'eau à l'équilibre de moins de 25 % ; et une absorption de solution aqueuse d'isopropanol de moins de 50 %.

2. Film polymère de la revendication 1 ayant une épaisseur comprise dans l'intervalle allant de 10 $\mu$m (0,0004 pouce) à 254 $\mu$m (0,01 pouce).

3. Film polymère de la revendication 1 ou 2, dans lequel le composant polymère de ladite seconde couche principale comprend un copolymère séquencé polyéther ester-amide.

4. Film polymère de la revendication 1 ou 2, dans lequel le composant polymère de ladite seconde couche comprend un polyéther uréthane.

5. Film polymère de la revendication 1 ou 2, dans lequel le composant polymère de ladite seconde couche comprend un polyester uréthane.

6. Film polymère de la revendication 1 ou 2, dans lequel le composant polymère de ladite seconde couche principale comprend un copolymère poly(éther-ester).

7. Structure stratifiée comprenant un premier tissu joint à l'une des surfaces principales du film bicomposant de l'une quelconque des revendications 1 à 6.

8. Structure stratifiée de la revendication 7, dans laquelle ledit tissu est un tissu tissé, un tissu tricoté ou un tissu non tissé.

9. Structure stratifiée de la revendication 8, dans laquelle ledit tissu est un tissu non tissé.

10. Structure stratifiée de la revendication 9, dans laquelle ledit tissu non tissé comprend des fibres de polyamide et est fixé par points.

11. Structure stratifiée de la revendication 9, dans laquelle ledit tissu non tissé comprend une toile hydroemmêlée de fibres de polyester.

12. Structure stratifiée des revendications 9 à 11, dans laquelle ledit tissu non tissé est ignifuge et est fixé à la seconde surface principale dudit film bicomposant.

13. Structure stratifiée de l'une quelconque des revendications 7 à 12, dans laquelle ledit premier tissu est joint audit film bicomposant par une couche discontinue d'adhésif.

**14.** Structure stratifiée de la revendication 13, dans laquelle ledit adhésif est un adhésif de polyuréthane durcissable à l'humidité.

**15.** Structure stratifiée de l'une quelconque des revendications 7 à 11, dans laquelle ledit premier tissu est joint à la première surface principale dudit film bicomposant.

**16.** Stratifié tricomposant comprenant un premier tissu non tissé, un second tissu non tissé et le film bicomposant de l'une quelconque des revendications 1 à 6, ledit premier tissu non tissé comprenant une couche de fibres synthétiques liées par de l'air chaud, ledit second tissu non tissé étant ignifuge et comprenant une toile hydroemmêlée de fibres de longueurs discontinues, ledit premier tissu non tissé étant joint à la première surface principale dudit film bicomposant et ledit second tissu non tissé étant joint à la seconde surface principale dudit film bicomposant.

*FIG-1*

*FIG-2*

FIG-3

FIG-4